# EUROPEAN PATENT SPECIFICATION

(11) **EP 4 566 657 B1**
(45) Date of publication and mention of the grant of the patent: **09.09.2026**
(21) Application number: 24217676.6
(22) Date of filing: 05.12.2024
(51) Int. Cl.: A61N 1/375

(54) **BIOSTIMULATOR HAVING SLIDABLE FRAME**
BIOSTIMULATOR MIT VERSCHIEBBAREM RAHMEN
BIOSTIMULATEUR AYANT UN CADRE COULISSANT

(30) Priority: 06.12.2023 US 202363606719 P; 22.11.2024 US 202418957571
(43) Date of publication of application: 11.06.2025
(73) Proprietor: Pacesetter, Inc., Sylmar, CA 91342 (US)
(72) Inventor: Li, Anthony J, Sylmar, CA 91342 (US); Sheth, Jay, Sylmar, CA 91342 (US)
(74) Representative: Barker Brettell Sweden AB

(56) References cited:
- US-A1- 2011 251 662
- US-A1- 2019 083 801
- US-A1- 2022 347 429

## Description

### TECHNICAL FIELD

The present disclosure relates to biostimulators. More specifically, the present disclosure relates to leadless biostimulators having tissue anchors.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Cardiac pacing by currently available or conventional pacemakers is usually performed by a pulse generator implanted subcutaneously or sub-muscularly in or near a patient's pectoral region. The generator usually connects to a proximal end of one or more implanted leads, the distal end of which contains one or more electrodes for positioning adjacent to the inside or outside wall of a cardiac chamber. Although more than one hundred thousand conventional cardiac pacing systems are implanted annually, various well-known difficulties exist, and there is an ongoing need for improvement in the art.

US 2019/083801 A1 discloses an implantable medical device having a housing with a proximal end, a distal end and an outer sidewall extending from the proximal end to the distal end. A fixation sheath includes a housing sheath portion extending along the outer sidewall of the housing, and a fixation member portion extending from the housing sheath portion. The housing sheath portion is advanceable from a first position along the outer sidewall of the housing in which the fixation member portion is retracted toward the proximal end of the housing to a second position along the outer sidewall of the housing in which the fixation member portion is deployed to extend away from the housing distal end for anchoring the implantable medical device at an implant site.

US 2011/251662 A1 discloses a fixation device for retaining a leadless medical implant to tissue includes an array of elongate tines having self-expanding distal portions. The fixation tines may be advanced between an implant body and an outer jacket to deploy the tines from a delivery configuration in which the tines are constrained by the outer jacket to an expanded configuration in which the distal end portions of the tines are released from the outer jacket. The implant and fixation device are contained within a sheath for delivery to the treatment site and a pusher within the sheath advances the fixation device relative to the implant body and deploys the tines. A distal end of the implant having an electrode may form a distal tip of the delivery system, and a potential implantation site may be tested prior to deployment of the fixation device to allow for easy repositioning of the implant.

### SUMMARY

Various embodiments, variations and examples are herein described for an implantable biostimulator, and components thereof which may include a housing, may include a frame which may be a specific type of frame, may include fixation tines which may be extensible and/or retractable, and may include an electrode connected with pacing circuitry.

The invention is defined in the independent claim. Further embodiments of the invention are defined in the dependent claims.

In one embodiment, an implantable biostimulator includes a housing and a frame. The housing includes an electronics compartment which contains pacing circuitry. The housing includes a longitudinal channel and one or more circumferential channels. The frame is movably engaged to the longitudinal channel and has one or more circumferential bands or rings each constrained in and engaging a respective circumferential channel. The frame has fixation tines at a distal end of the frame. The longitudinal channel has clearance to allow the frame to move circumferentially within the longitudinal channel when the one or more circumferential bands or rings rotate relative to the housing within the one or more circumferential channels.

In one embodiment, a biostimulator system may include a biostimulator transport system and a biostimulator. The biostimulator transport system may have a handle and an elongated catheter extending from the handle to a distal catheter end. The biostimulator may be releasably mounted or releasably mountable on the distal catheter end of the transport system. The biostimulator may have a housing. The biostimulator may have a frame movably engaged to a longitudinal guide of the housing. The biostimulator may have fixation tines at a distal end of the frame.

The above summary does not include an exhaustive list of all aspects of the present invention. It is contemplated that the invention includes all devices, and systems, that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIGS. 1A-1C depicts stages of extraction and fixation retraction of a leadless implantable medical device that has extensible, retractable fixation tines, in accordance with an embodiment.
FIG. 1A depicts a leadless implantable medical device with fixation tines extended, as for implantation, prior to retrieval.
FIG. 1B depicts a leadless implantable medical device with fixation tines partially retracted, as for retrieval.
FIG. 1C depicts a leadless implantable medical device with fixation tines retracted, as for retrieval.
FIGS. 2A-2D depict external and internal views of an embodiment of a leadless implantable medical device with extensible, retractable fixation tines.

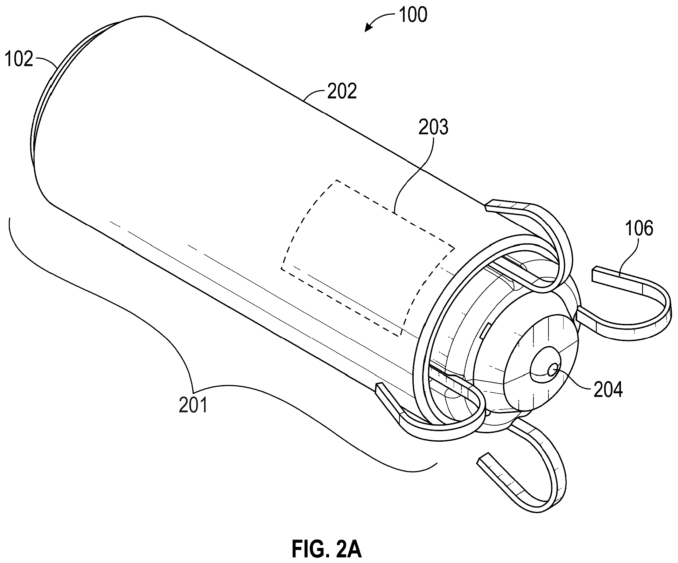

FIG. 2A is a perspective external view of a leadless implantable medical device, with the fixation tines extended, in an embodiment.

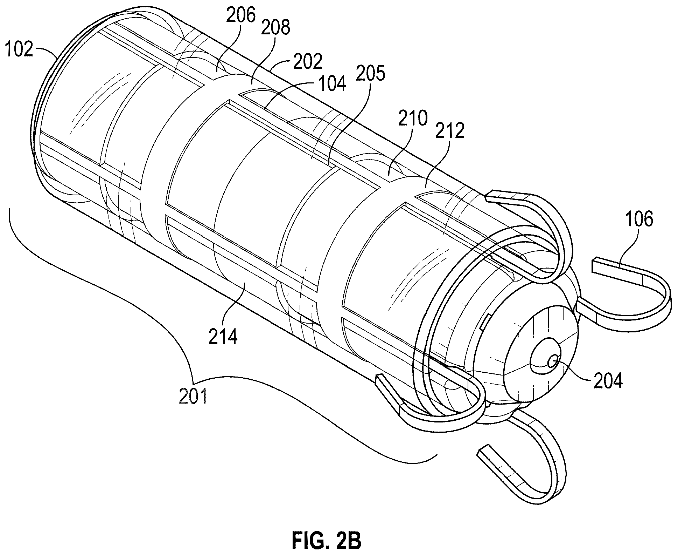

FIG. 2B is a perspective internal view of a leadless implantable medical device, with the fixation tines extended, in an embodiment.

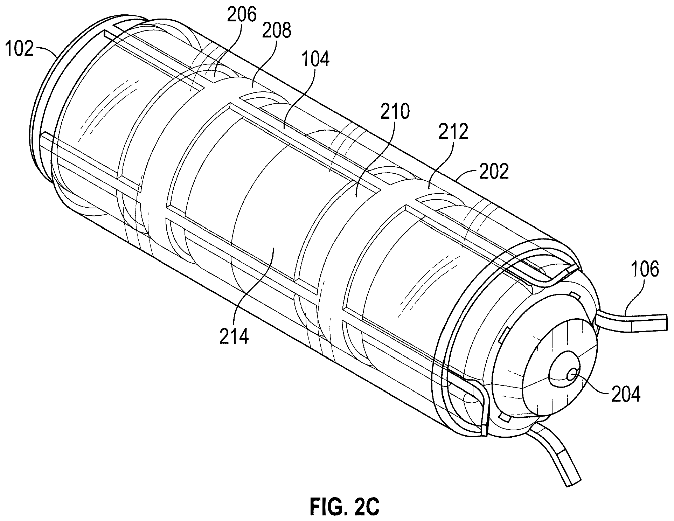

FIG. 2C is a perspective internal view of a leadless implantable medical device, with the fixation tines partially extended or partially retracted, in an embodiment.

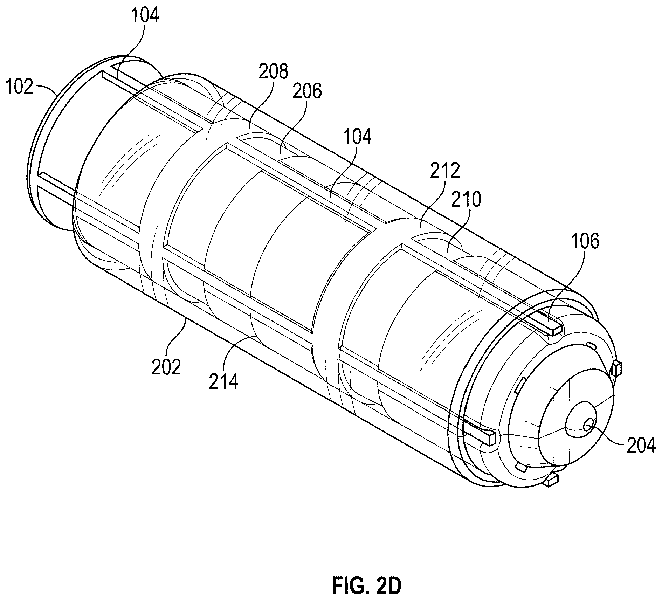

FIG. 2D is a perspective internal view of a leadless implantable medical device, with the fixation tines retracted, in an embodiment.

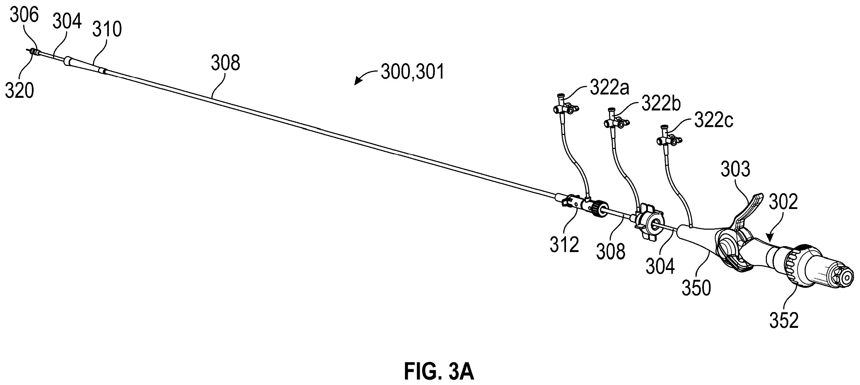

FIGS. 3A-3B are perspective views of a biostimulator delivery system, in accordance with an embodiment.

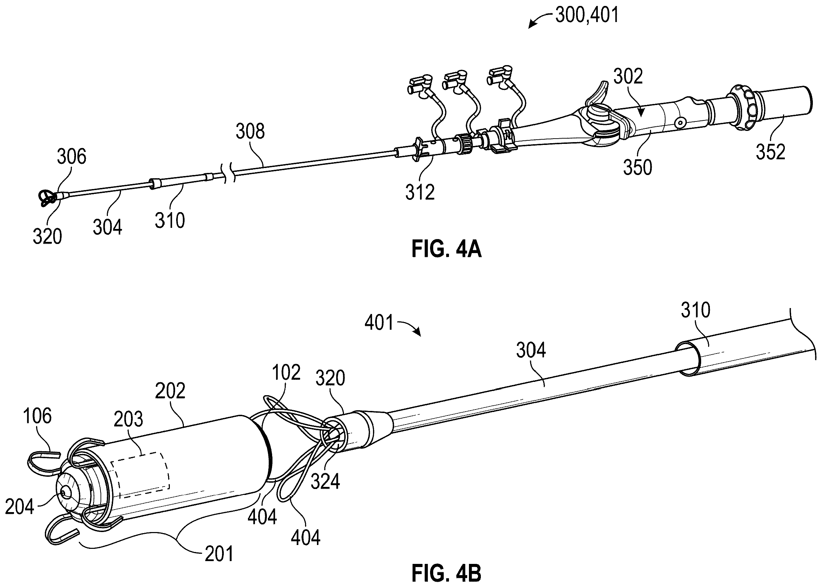

FIGS. 4A-4B are perspective views of a biostimulator retrieval system, in accordance with an embodiment.

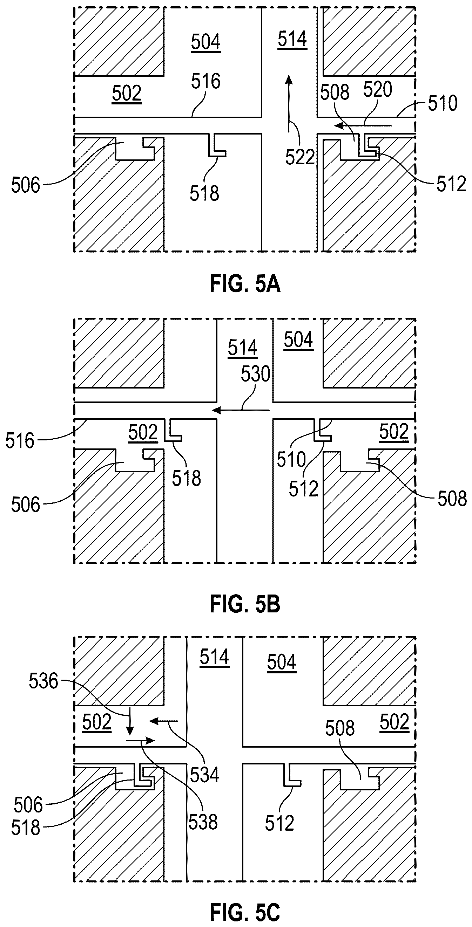

FIGS. 5A-C depict a locking mechanism in stages of locking, unlocking, and locking in a leadless implantable medical device, in accordance with an embodiment.
FIG. 5A depicts the frame with a locking tab engaging a locking channel, which locks the frame in a locked position with the fixation tines extended.
FIG. 5B depicts the frame with locking tabs disengaged from locking channels, as may occur with the frame in an unlocked position between locking positions, with the fixation tines partially extended or partially retracted.
Fig. 5C depicts the frame with another locking tab engaging another locking channel, which locks the frame in a further locked position with the fixation tines retracted.

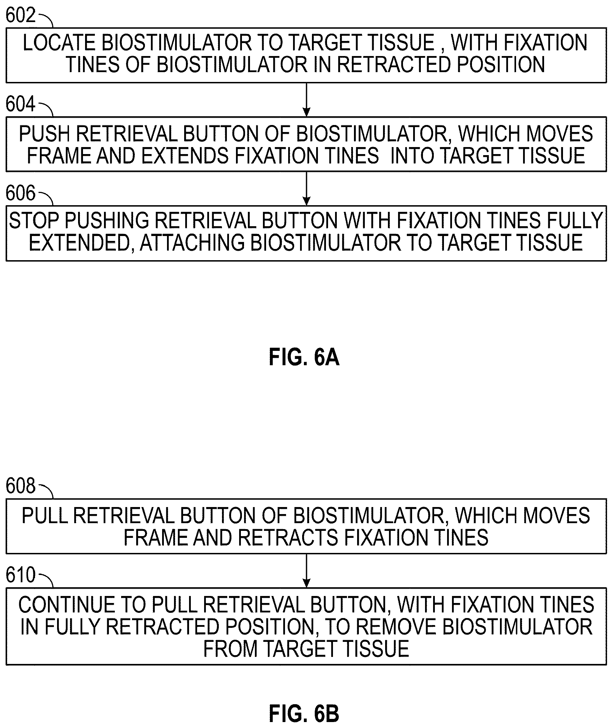

FIG. 6A-6B is a flow diagram depicting a method of treatment using an implantable biostimulator, which may be practiced using embodiments of a leadless implantable medical device described herein.
FIG. 6A depicts a method of treatment using an implantable biostimulator, to implant the biostimulator.
FIG. 6B depicts a method of treatment using an implantable biostimulator, to remove the biostimulator from implantation.

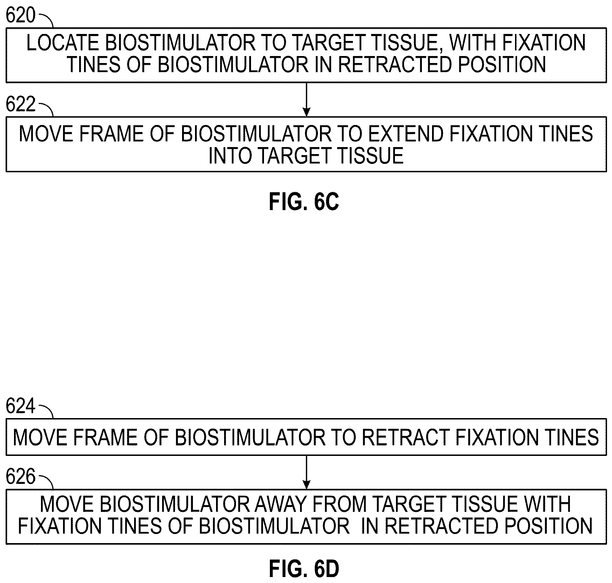

FIG. 6C-6D is a further flow diagram depicting a further method of treatment using an implantable biostimulator, which may be practiced using embodiments of a leadless implantable medical device described herein.
FIG. 6C depicts a method of treatment using an implantable biostimulator, to implant the biostimulator.
FIG. 6D depicts a method of treatment using an implantable biostimulator, to remove the biostimulator from implantation.

### DETAILED DESCRIPTION

Described herein are embodiments of a biostimulator, which may be a leadless implantable medical device, a leadless cardiac pacemaker, or leadless biostimulator for implantation to stimulate biological tissue, such as nerves or muscle, for example heart tissue, brain tissue, nerve bundles, or muscle tissue. Transport systems, for use in implantation and retrieval of the biostimulator, including a biostimulator delivery system and a biostimulator retrieval system, are described. The biostimulator transport system can be used to deliver or retrieve a biostimulator, e.g., a cardiac pacemaker, from a heart of a patient. The biostimulator may, however, be used in other applications, such as deep brain stimulation. Thus, reference to the biostimulator as being a cardiac pacemaker may be not limiting.

Embodiments of a biostimulator feature fixation tines that are extensible and retractable. Some embodiments feature a locking mechanism that locks the fixation tines in an extended position, for implantation to a tissue, and/or locks the fixation tines in a retracted position, for retrieval and/or pre-implantation. The terms "fully" and "partially" are relative to a given embodiment and capabilities thereof. For example, "fully retracted" fixation tines are more retracted than "partially retracted" fixation tines, and "fully extended" fixation tines are more extended than "partially extended" fixation tines.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative portion of an element, a relative position, or relative direction. For example, "distal" may indicate a first direction along a central axis of a biostimulator or a biostimulator transport system. Similarly, "proximal" may indicate a second direction opposite to the first direction. Such terms are provided to establish relative frames of reference, however, and are not intended to limit the use or orientation of a biostimulator or a biostimulator transport system to a specific configuration described in the various embodiments below.

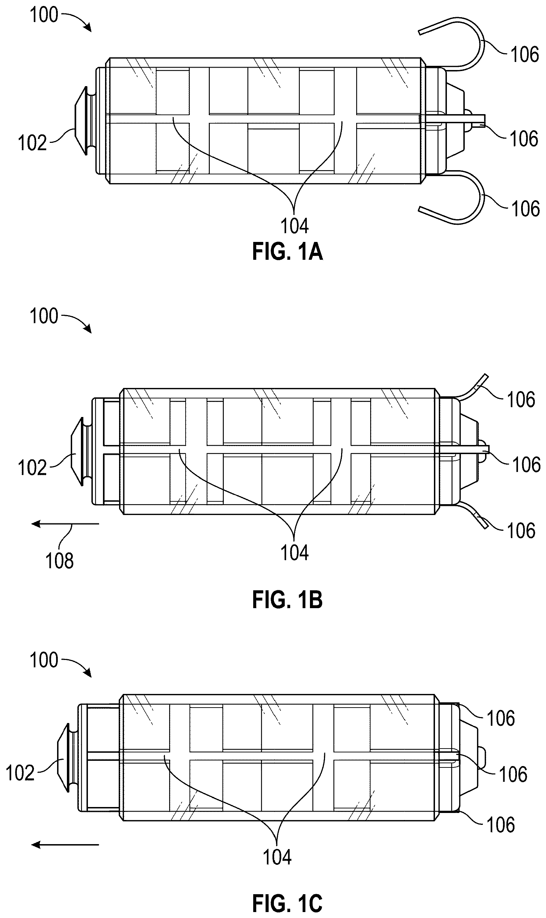

FIGS. 1A-1C depicts stages of extraction and fixation retraction of a leadless implantable medical device that has extensible, retractable fixation tines 106, in accordance with an embodiment. The drawings may be viewed in one sequence, FIGS. 1A, 1B, 1C to show retrieval with the fixation tines 106 retracting, and another sequence, FIGS. 1C, 1B, 1A to show implantation with the fixation tines 106 extending.

FIG. 1A depicts a leadless implantable medical device with fixation tines extended, as for implantation, prior to retrieval. Or, the fixation tines 106 are fully extended and grasping a tissue (not shown, but readily understood), at the end of an implantation sequence. In operation for retrieval, a retrieval button 102 or a frame 104 may be grasped and pulled (e.g., by a biostimulator transport system or a biostimulator retrieval system, see FIG. 4A-4B). Pulling the retrieval button 102 or the frame 104 away from the proximal end of the main body of the biostimulator 100 or in a proximal direction relative to the transport system or retrieval system, slides the frame 104 in the proximal direction, which retracts the fixation tines 106 from the extended position shown in FIG. 1A. In the embodiment shown, the retrieval button 102 may be at the proximal end of the frame 104, the fixation tines 106 are at the distal end of the frame, and the retrieval button 102, frame 104, and fixation tines 106 form a unitary body that may be longitudinally movable to and fro, or in a proximal direction and a distal direction, relative to the remainder of the biostimulator 100. In some embodiments, the unitary body including the retrieval button 102, frame 104 and fixation tines 106 may be of a memory material, which may be nitinol (nickel titanium alloy). In some embodiments, the fixation tines 106 are termed fully extended, or in an extended or extension position. The frame 104 may be in an extension position, or a fixation tines extended position.

FIG. 1B depicts a leadless implantable medical device with fixation tines 106 partially retracted, as for retrieval. Or, the fixation tines 106 are partially extended, as for implantation. From pulling the retrieval button 102, for extraction of the biostimulator 100, or pushing the retrieval button 102, for implantation of the biostimulator 100, the frame 104 may be in a middle position relative to the remainder of the biostimulator 100. Alternatively, the frame 104 may be in a fixation tines partially retracted position, or a fixation tines partially extended position, which may be relative to an implantation sequence or a retrieval sequence.

FIG. 1C depicts a leadless implantable medical device with fixation tines 106 retracted, as for retrieval. Or, the fixation tines 106 are retracted, for implantation. From pulling the retrieval button 102, for extraction, the biostimulator 100 may be pulled in a proximal direction away from the tissue to which the biostimulator 100 was implanted. Or, from pushing the retrieval button 102, for implantation, the biostimulator 100 may be contacting tissue, and prepared for extension of the fixation tines 106. In some embodiments, the fixation tines 106 are termed fully retracted, or in a retracted position. The frame 104 may be in a retracted position, or a fixation tines retracted position.

FIGS. 2A-2D depict external and internal views of an embodiment of a leadless implantable medical device with extensible, retractable fixation tines 106. Further details of external and internal structure are described below, for an embodiment of the biostimulator 100.

FIG. 2A is a perspective external view of a leadless implantable medical device, with the fixation tines 106 extended, in an embodiment. A battery and electronics are housed in the main body of the biostimulator 100, which may be termed a housing 201 and includes an outer cylindrical can 202 or casing enclosing an inner cylindrical can or casing (see FIGS. 2B-2D). More particularly, the housing 201 can include an electronics compartment 203 (represented by dotted lines) containing pacing circuitry. The retrieval button 102 may be at the proximal end of the cylindrical can 202, in a fixation tines extended position, and the fixation tines 106 are at the distal end of the cylindrical can 202, in an extended position or fixation tines extended position. An electrode 204, at the distal end of the cylindrical can 202, or the distal end of the main body of the biostimulator 100, may be electrically connected to electronics within the biostimulator 100, and may be for contact to tissue with the biostimulator 100 implanted.

FIG. 2B is a perspective internal view of a leadless implantable medical device, with the fixation tines extended, in an embodiment. Here, the outer cylindrical can 202 is depicted as see-through, so that internal structure is visible. The frame 104, which has the retrieval button 102 at a proximal end, and the fixation tines 106 at a distal end, has in this embodiment longitudinal members each ending in a respective fixation tine 106 at the distal end of the frame 104. The housing can include a longitudinal guide 205, within which the frame 104 is movably engaged. For example, in this and some other embodiments, the frame 104 may be slidably sandwiched or movably located within the housing 201, more specifically sandwiched or located between the outer cylindrical can 202 and the inner cylindrical can 214 of the housing 201 so as to be slidable or movable relative thereto. In an embodiment, the longitudinal guide 205 of the housing 201 could comprise longitudinal tracks in an outer surface of the inner cylindrical can 214. In such a case, the frame 104 could be sandwiched between the outer cylindrical can 202 and the inner cylindrical can 214, slidably engaging the longitudinal tracks. The frame 104 further includes one or more circumferential bands or rings 208, 212, which tie together the longitudinal members and the fixation tines 106 lending structural strength in some embodiments. The circumferential rings 208, 212 are each slidable in a longitudinal direction, to and fro or in a proximal direction and a distal direction, each constrained in and engaging a respective circumferential channel 206, 210. Here, one ring 212 may be slidable in a respective circumferential channel 210, and another ring 208 may be slidable in another, respective circumferential channel 206. The circumferential channel or channels 206, 210 act as stops to constrain forward and backward motion of the frame 104, which constrains forward and backward motion of the retrieval button 102 at the proximal end of the frame 104, and constrains forward and backward motion, i.e., extension and retraction, of the fixation tines 106. In FIG. 2B, each ring 208, 212 may be at a forward or distal extreme of the respective circumferential channel 206, 210, constraining the full extension of the fixation tines 106. With full extension of the fixation tines 106, the tissue to which the biostimulator 100 may be attached by the fixation tines 106 may receive stimulation from the electrode 204. In further embodiments, other stops, arrangements of stops, and types of stops that constrain movement of the frame and thereby constrain movement of the fixation tines 106 are readily devised.

In various embodiments, the longitudinal members of the frame 104 engage and slide in longitudinal channels of the main body of the biostimulator 100, and these longitudinal channels may be grooves in the inner can 214 as shown here, or grooves in the outer can 202 of the housing 201, or both. The cylindrical rings 208, 212 engage and slide in circumferential channels 206, 210, which may be grooves in the inner can 214, as shown here, or grooves in the outer can 202 of the housing 201, or both, in further embodiments. See also FIGS. 5A-5C for further details of channels, albeit with a locking mechanism.

FIG. 2C is a perspective internal view of a leadless implantable medical device, with the fixation tines 106 partially extended or partially retracted, in an embodiment. The circumferential rings 208, 212 of the frame 104 are each at a middle position, or fixation tines partially extended or partially retracted position, relative to the circumferential channels 206, 210. In turn, through connection to the frame 104, the retraction button 102 may be at a partially extended or partially depressed position.

FIG. 2D is a perspective internal view of a leadless implantable medical device, with the fixation tines 106 retracted, in an embodiment. The circumferential rings 208, 212 of the frame 104 are at a fixation tines retracted position relative to the circumferential channel 206, 208. In turn, through connection to the frame 104, the retraction button 102 may be in a fully extended position, or fully withdrawn from the main body (or the outer cylindrical can 202 or inner cylindrical can 214).

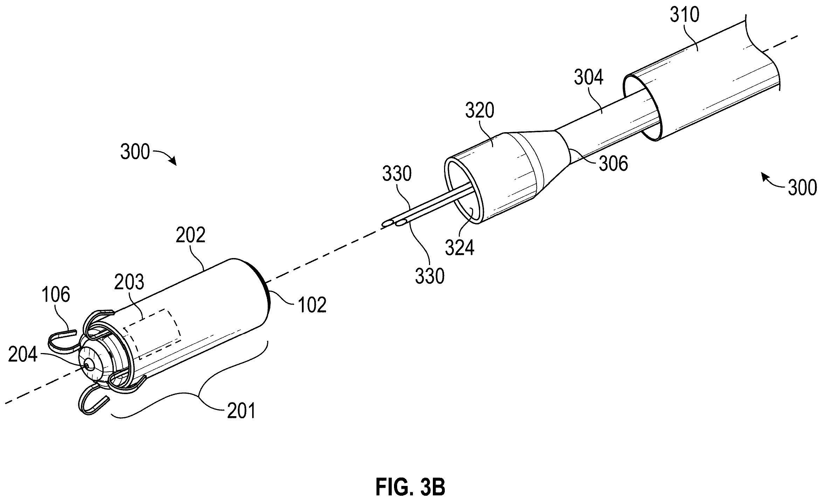

FIGS. 3A-3B and 4A-4B depict suitable biostimulator transport systems which may be used for implantation and retrieval of the biostimulator 100, in various embodiments. Although described with reference to a different type of biostimulator, it should be appreciated that the biostimulator delivery system of FIGS. 3A and 3B and the biostimulator retrieval system of FIGS. 4A and 4B may be usable with various biostimulators, including embodiments of the biostimulator 100 described herein. It is understood that engagement features of tools and embodiments of the biostimulator 100 should be arranged for engagement of the biostimulator 100, operation of the release button thereof, release of the biostimulator 100, and retrieval of the biostimulator 100, with the chosen tool, e.g., biostimulator delivery system or biostimulator retrieval system.

It should be appreciated that, in various embodiments, there may be two distinct tools, one for implantation and one for retrieval, a single tool used for both implantation and retrieval, or a single tool with swappable attachments, one for implantation and one for retrieval. Thus, a biostimulator transport system may be or include biostimulator delivery system. A biostimulator transport system may be or include a biostimulator retrieval system. For example, a biostimulator transport system may have tether(s) that also function as snare(s), or vice versa, and thus may be usable as a biostimulator delivery system and a biostimulator retrieval system. A biostimulator transport system may have swappable attachments, one of which has tether(s), another of which has snare(s). With the tether(s) attachment, the biostimulator transport system may be operable as a biostimulator delivery system. With the snare(s) attachment, the biostimulator transport system may be operable as a biostimulator retrieval system.

Referring to FIG. 3A, a perspective view of a biostimulator transport system is shown in accordance with an embodiment. A biostimulator transport system 300 may be used for delivery and/or retrieval of the biostimulator 100, e.g., a leadless pacemaker, into or from a patient. For example, the biostimulator transport system 300 can be or include a biostimulator delivery system 301 used for delivery of the biostimulator 100 into a patient.

The biostimulator transport system 300 can include a handle 302, and an elongated catheter 304 extending distally from the handle 302 to a distal catheter end 306. The handle 302 can include several portions, e.g., a distal handle portion 350 and a proximal handle portion 352, and features that allow a user to provide inputs at a proximal end of the system that translate to outputs at the distal end of the system. For example, the elongated catheter 304 can be a deflectable catheter, and an operator can use the handle 302 to steer the distal catheter end 306 in the patient.

In an embodiment, the handle 302 includes a deflection lever 303 that can be used to deflect the distal catheter end 306. By pivoting the deflection lever 303 toward a distal handle portion 350 of the handle 302, the operator can cause a pull ring assembly extending within the elongated catheter 304 to apply off-axis compression to the elongated catheter 304, resulting in lateral deflection of the distal catheter end 306.

The handle 302 can be used to apply a torque to a docking cap 320 at the distal end of the system. In an embodiment, the proximal handle portion 352 can be rotationally and/or longitudinally moveable relative to the distal handle portion 350. For example, the distal handle portion 350 can be coupled to the elongated catheter 304 and the proximal handle portion 352 can be coupled to a torque shaft extending within the elongated catheter 304. The docking cap 320 can be mounted on the torque shaft. Accordingly, an operator can rotate the proximal handle portion 352 relative to the distal handle portion 350 to impart torque to the torque shaft. The torque can cause the docking cap 320, which may be rotationally linked to the proximal handle portion 352 through the torque shaft to rotate relative to the elongated catheter 304, which may be rotationally linked to the distal handle portion 350.

In an embodiment, the biostimulator transport system 300 includes a protective sheath 308 mounted on the elongated catheter 304. The protective sheath 308 can be slidably disposed on the elongated catheter 304. The protective sheath 308 can include an atraumatic end 310, e.g., a soft, funnel-shaped distal portion, that can slide distally over the distal catheter end 306 of the elongated catheter 304 and/or the biostimulator 100 (not shown). The atraumatic end 310 can have an outer dimension, which may be larger than a proximal portion of the protective sheath 308. For example, the atraumatic end 310 may flare in a distal direction to a funnel opening that can advance over a docking cap 320 of the biostimulator transport system 300. An outer dimension of the atraumatic end 310 can be larger than a region of the protective sheath 308 supporting a valve bypass tool 312.

The valve bypass tool 312 can be slidably disposed on the protective sheath 308 such that a distal portion of the valve bypass tool 312 can slide distally over the distal catheter end 306 of the elongated catheter 304 and/or the atraumatic end 310 of the protective sheath 308. More particularly, the valve bypass tool 312 can be inserted into an access introducer to gain access to the patient vasculature, and after access is established, the distal portion of the protective sheath 308 and/or the distal end of the elongated catheter 304 can be advanced through the valve bypass tool 312 into the patient.

The valve bypass tool 312, the protective sheath 308, and the elongated catheter 304 can have respective flush ports 322a, 322b, and 322c extending respectively therefrom. Each of the longitudinal bodies are displaceable proximal-distal relative to each other, and thus, the flush ports can be used to introduce and/or flush saline or other fluids between the longitudinal bodies or through the respective components in different relative positions.

Referring to FIG. 3B, a distal perspective view of a biostimulator transport system 300 having a docking cap to receive a biostimulator is shown in accordance with an embodiment. The distal catheter end 306 of the elongated catheter 304 may be selectively connectable to the biostimulator 100. More particularly, the biostimulator 100 can be mounted on the distal catheter end 306 of the elongated catheter 304. In an embodiment, the biostimulator 100 can be mounted to the biostimulator transport system using an attachment feature of the biostimulator 100, for example the retrieval button 102 includes a channel (not shown) shaped and sized to receive one or more tethers 330, or may be otherwise shaped and/or sized to receive one or more tethers 130. The tethers 330 can comprise wires, shafts, tubes, cords, ropes, strings, or other similar structures that can extend throughout the catheter shaft 304. For example, the tethers 330 can extend through a shaft lumen of a torque shaft assembly. In some embodiments, the tethers 330 comprise a shape memory material, such as nickel-titanium. In other embodiments, the tethers 330 comprise stainless steel wires or braids. The tethers 330 can be inserted into and locked within the attachment feature, e.g., the retrieval button 102 to connect the biostimulator 100 to the biostimulator transport system 300.

When the tethers 330 are locked within the attachment feature 218, the tethers 330 can be retracted to pull the biostimulator 100 toward the docking cap 320. The docking cap 320 can include a docking cavity 324 having a shape and size to receive the attachment feature, e.g., retrieval button 102 of the biostimulator 100. As the biostimulator 100 moves toward the docking cap 320, the attachment feature, e.g., the retrieval button 102 can insert into the docking cavity 324. Accordingly, the docking cavity 324 can receive the attachment feature, e.g., retrieval button 102 to dock the biostimulator 100 to the biostimulator transport system 300 for delivery to the patient.

Torque can be transmitted from the docking cap 320 to the biostimulator 100 via the torque shaft when the attachment feature, e.g., the retrieval button 102 may be received in the docking cap 320. More particularly, the torque shaft can be rotated in a first direction, e.g., clockwise, to transmit torque through the docking cap 320 to the attachment feature, e.g., the retrieval button 102, and to cause the frame 104 to rotate relative to the housing.

The biostimulator 100 can be protected by the atraumatic end 310 of the protective sheath 308 during delivery and/or retrieval of the biostimulator 100 from the patient. The atraumatic end 310 can have a braided or woven tubular construction. The atraumatic end 310 can therefore be advanced over the biostimulator 100 and may expand radially over the biostimulator 100 in the case where an outer dimension of the biostimulator 100 may be greater than the inner diameter of the atraumatic end 310. Accordingly, the atraumatic end 310 can cover the biostimulator 100 to protect the biostimulator during advancement into the patient.

Referring to FIG. 4A, a perspective view of a biostimulator retrieval system is shown in accordance with an embodiment. The biostimulator transport system 300 may be or include a biostimulator retrieval system 401. The biostimulator retrieval system 401 can be used to explant a biostimulator 100 from the atrium and/or the ventricle of the heart of the patient. Removal and retrieval of the biostimulator(s) 100 may be accomplished endocardially. For example, the torque shaft of the elongated catheter 304 can be pulled and/or rotated, or combinations thereof, to disengage the biostimulator 100 from the heart tissue. Accordingly, retrieval system 401 shown in FIG. 4A can have a structure similar to that shown and described with respect to the delivery system of FIG. 3A to retrieve the biostimulator 100 from a target anatomy. The similarly numbered components of the biostimulator retrieval system 401 are not described again here for brevity.

Referring to FIG. 4B, a perspective view of a distal portion of a biostimulator retrieval system prior to attaching to a biostimulator is shown in accordance with an embodiment. The distal portion of the biostimulator transport system 300 can include features to engage the leadless pacemaker to facilitate capturing and unscrewing the biostimulator 100 from the target tissue. More particularly, the biostimulator retrieval system 401 can include a snare 402 extending through the elongated catheter 304 to grasp a biostimulator 100 or other medical device, for example by grasping the retrieval button 102 of the biostimulator 100. The snare 402 can include at least one snare loop 404, e.g., a wire loop, extending from the elongated catheter 304. As the snare 402 may be advanced distally out of the biostimulator transport system 300 from the docking cap 320, the loop(s) 404 can expand in size to aid a user in positioning the snare 402 around or in proximity to the biostimulator 100 to be retrieved. In some implementations, as in FIG. 4B, the snare 402 can include multiple loops 404, such as three loops. However, any number of loops can be used as long as the elongated catheter 304 contains sufficient volume to accommodate the loops.

The distal portion of the retrieval catheter can include the docking cap 320 configured to allow docking of the leadless pacemaker with the biostimulator transport system 300 after engaging the pacemaker with the snare 402. A user can transmit torque through the torque shaft via handle 302 to rotate the docking cap 320 relative to the elongated catheter 304. More particularly, the torque shaft can extend through the length of the catheter to the handle 302, e.g., the proximal handle portion 352, which may be coupled to the torque shaft. Rotation or actuation of the handle 302 rotates the torque shaft, thereby rotating the docking cap 320 at the end of the retrieval catheter. The protective sheath 308 can be positioned along the elongated catheter 304, and can be advanced or retracted to cover or expose the docking cap 320 and the leadless pacemaker 100 using the atraumatic end 310.

During retrieval, the biostimulator transport system 300 can be navigated through the patient to the implant site. The snare 402 can be placed over the attachment feature, e.g., retrieval button 102 and the loops of the snare 420 can be reduced in size, thereby grasping or locking onto the attachment feature, e.g., retrieval button 102 of the biostimulator 100. Following capture and locking of the snare 402 with the retrieval button 102, the biostimulator 100 may be docked within the docking cap 320. More particularly, the attachment feature, e.g. retrieval button 102 of the biostimulator 100 can be pulled into a docking cavity 324 of the docking cap 320. In some implementations, the docking cap 320 can include a key or interference feature configured to mate with and engage a corresponding key or feature on the biostimulator 100. In some implementations, the key or slot on the docking cap 320 can match a unique shape or feature of the attachment feature, e.g., retrieval button 102 of the biostimulator 100. Because the key or slot on or in the docking cap 320 can mate with and engage the key or slot on the biostimulator 100, the retrieval catheter can be configured to apply torque to the retrieval button 102 (e.g., for locking and/or unlocking, see FIGS. 5A-C) and/or can be configured to apply pulling to the retrieval button 102 (e.g., for retracting fixation tines 106 and/or moving the biostimulator 100 away from target tissue).

FIGS. 5A-C depict a locking mechanism in stages of locking, unlocking, and locking in a leadless implantable medical device, in accordance with an embodiment. The drawings may be viewed in one sequence, FIGS. 5A, 5B, 5C to show locked and unlocking from a locked position of the frame with the fixation tines fully extended, transition between locking positions, and locking and locked to a further locked position of the frame with the fixation tines fully retracted. These drawings may be viewed in another sequence, FIGS. 5C, 5B, 5A to show locked and unlocking from a locked position of the frame with the fixation tines retracted, transition between locking positions, and locking and locked to a further locked position of the frame with the fixation tines extended. FIGS. 5A-C show a close up view of a portion of the frame, a longitudinal channel 502 and a circumferential channel 504, which can be further understood with reference to Figs. 2A-D, albeit there shown without a locking mechanism. It should be appreciated that variations with other numbers of channels, other numbers of locking tabs and locking channels, other shapes of locking tabs and locking channels, other orientations, and further embodiments with pins and grooves, detents, other locking shapes, etc., with related functionality are readily developed in keeping with the teachings herein.

FIG. 5A depicts the frame with a locking tab 512 engaging a locking channel 508, which locks the frame in a locked position with the fixation tines extended (see FIGS. 1A, 2A, 2B). Another locking tab 508 is shown disengaged from a target locking channel 506, with which engagement is shown in Fig. 5C. To unlock the locking tab 512 from the locking channel 508, the frame 516 may be moved in a proximal direction 520 or longitudinal direction, as may be accomplished by pulling the retraction button 102 in a proximal direction (see, e.g., FIG. 1A), for example using an extraction system. Then, the frame 516 may be moved in a counterclockwise circumferential direction 522 or a lateral direction, as may be accomplished by rotating the retraction button 102 in a counterclockwise direction (see, e.g., FIG. 1A and visualize counterclockwise rotation of the retraction button 102 relative to the proximal end of the biostimulator 100). These two motions may disengage the locking tab 512 from the locking channel 508, positioning the portion 510 of a longitudinal member of the frame at the upper or far side of the longitudinal channel 502 (see FIG. 5B).

FIG. 5B depicts the frame with locking tabs 512, 518 disengaged from locking channels 508, 506, as may occur with the frame in an unlocked position between locking positions, with the fixation tines partially extended or partially retracted (see FIGS. 1B, 2C). With the locking tabs 512, 518 freed up from respective locking channels 508, 506, and the portions 510, 516 of the frame sliding along the longitudinal channel 502, the frame may be moved in a proximal longitudinal direction 530, as may be accomplished by pulling the retraction button 102 in the transition between FIG. 5A and FIG. 5C, or pushing the retraction button 102 in the transition between FIG. 5C and FIG. 5A. In order to provide sufficient clearance for the locking tabs 512, 518 and frame to move freely, the longitudinal channel 502 should be sufficiently wide (e.g., wider than for a closer sliding channel fit in embodiments without the locking tabs 512, 518). Also, the circumferential channel 504 should be sufficiently wide, as in other embodiments, to support longitudinal movement of the frame and constrain the frame as to the extension and retraction of the fixation tines, and also relative to the members of the locking feature, thus supporting locking in two different positions, unlocking, and motion therebetween.

Fig. 5C depicts the frame with another locking tab 518 engaging another locking channel 506, which may lock the frame in a further locked position with the fixation tines retracted (see FIG. 1C and FIG. 2D). From the position shown in FIG. 5B, the frame may be further moved or slid along the longitudinal channel 502, in the proximal longitudinal direction 534 for example until the circumferential ring 514 meets one proximal boundary of the circumferential channel 504. Then the frame may be moved in a clockwise circumferential direction 536, as may be accomplished by rotating the retraction button 102 in a clockwise direction (see FIG. 1C or FIG. 2D, to visualize), which may engage the locking tab 518 into the locking channel 506. To complete the locking, in this embodiment, the frame may be moved in a distal longitudinal direction 538, as may be accomplished by pushing the retraction button 102 towards the main body of the biostimulator 100. Such action may lock the locking tab 518 in the locking cavity 506, while the other locking tab 512 remains disengaged from the respective locking channel 508.

FIGS. 6A-6B is a flow diagram depicting a method of treatment using an implantable biostimulator, which may be practiced using embodiments of a leadless implantable medical device described herein.

FIG. 6A depicts a method of treatment using an implantable biostimulator, to implant the biostimulator. The implantable biostimulator has extensible fixation tines, in an embodiment.

In an action 602, the biostimulator is located to a target tissue, with fixation tines of the biostimulator in a retracted position, in an embodiment. This action 602 may be performed using a biostimulator delivery system (see FIGS. 3A-3B).

In an action 604, the frame is moved to extend the fixation tines into the target tissue, in an embodiment. Pushing the retrieval button or the frame may be performed using a biostimulator delivery system. In one embodiment, a docking cap of the biostimulator delivery system is used for pushing the retrieval button of the biostimulator. In one embodiment, the docking cap is also used for rotating the retrieval button or the frame of the biostimulator, in an embodiment that has a locking mechanism.

In an action 606, movement of the retrieval button can be stopped with the fixation tines fully extended, attaching the biostimulator to the target tissue, in an embodiment. The biostimulator may be ejected from a protective sheath of the biostimulator delivery system, or otherwise released from the biostimulator delivery system.

Fig. 6B depicts a method of treatment using an implantable biostimulator, to remove the biostimulator from implantation. The implantable biostimulator has retractable fixation tines.

In an action 608, the frame of the biostimulator is moved to retract the fixation tines, in an embodiment. Pulling the retrieval button or the frame may be performed using a biostimulator retrieval system (see FIGS. 4A-4B). In one embodiment, a snare of a retrieval system is used for pulling the retrieval button of the biostimulator. In one embodiment, the snare of a retrieval system is also used for rotating the retrieval button or the frame of the biostimulator in an embodiment that has a locking mechanism.

In an action 610, the retrieval button or the frame is pulled until the fixation tines are in the fully retracted position, in an embodiment. The biostimulator is retracted from the target tissue, in an embodiment.

FIG. 6C-6D is a further flow diagram depicting a further method of treatment using an implantable biostimulator, which may be practiced using embodiments of a leadless implantable medical device described herein. It should be understood that the frame, of some embodiments, may be a specific type of frame as illustrated and that further frames with integral or attached fixation tines are readily developed in keeping with the teachings herein. For example, a frame may be a supportive structure and may have longitudinal struts, one or more lateral tabs which may be circumferential band(s), and/or a base that interconnects longitudinal struts. For example, a cylindrical frame, a hollow frame, latticework frame, a flat frame, a filled geometry frame, etc., with fixation tines, are possible. It should be understood that various mechanisms for engaging, moving, guiding and constraining movement of the frame of the biostimulator are readily developed in keeping with the teachings herein. For example, channels, grooves, slots, tracks, guides, etc. may be used, as may runners, wheels, pins, prongs, tabs, followers, sliders, etc. It should be understood that there may be further mechanisms and ways for moving the frame, which moves the fixation tines that are attached to or integral with the frame. Such components, mechanisms and functionality are generalized in the following embodiments, and further embodiments as readily developed.

FIG. 6C depicts a method of treatment using an implantable biostimulator, to implant the biostimulator. The biostimulator has extensible fixation tines attached to or integral with a frame. The fixation tines may be in a retracted position, or in an extended position, relative to a remainder of the biostimulator, e.g., relative to a housing of the biostimulator.

In an action 620, the biostimulator is located to a target tissue, with fixation tines of the biostimulator in a retracted position, in an embodiment.

In an action 622, the frame of the biostimulator is moved, to extend the fixation tines into the target tissue, in an embodiment. Action 622 may move the fixation tines to an extended position, attaching the biostimulator to the target tissue.

FIG. 6D depicts a method of treatment using an implantable biostimulator, to remove the biostimulator from implantation.

In an action 624, the frame of the biostimulator is moved, to retract the fixation tines, in an embodiment. Action 624 may move the fixation tines to a retracted position and may remove the fixation tines from the target tissue.

In an action 628, the biostimulator is moved away from the target tissue, with the fixation tines of the biostimulator in a retracted position, in an embodiment. Action 628 may retrieve the biostimulator from implantation.

In the foregoing specification, the invention has been described with reference to specific exemplary embodiments thereof. It will be evident that various modifications may be made thereto, which fall under the scope of the invention as set forth in the following claims. The specification and drawings are, accordingly, to be regarded in an illustrative sense rather than a restrictive sense.

## Claims

1. A biostimulator (100), comprising:
a housing (201) including an electronics compartment (203) containing pacing circuitry,
wherein the housing (201) includes a longitudinal channel (205) and one or more circumferential channels (206, 201); and
a frame (104) movably engaged to the longitudinal channel (205) and having one or more circumferential bands or rings (208, 212) each constrained in and engaging a respective circumferential channel (206, 210), wherein the frame (104) has a plurality of fixation tines (106) at a distal end of the frame (104), and the longitudinal channel (205) has clearance to allow the frame (104) to move circumferentially within the longitudinal channel (205) when the one or more circumferential bands or rings (208, 212) rotate relative to the housing (201) within the one or more circumferential channels (206, 210).

2. The biostimulator of claim 1, wherein the housing (201) has a plurality of stops (206, 210).

3. The biostimulator of claim 2, wherein the frame (104) is arranged to engage the plurality of stops (206, 210) so as to constrain extension and retraction of the fixation tines (106).

4. The biostimulator of any one of claims 1 to 3, wherein the housing (201) has an outer cylindrical can (202), and an inner cylindrical can (214) having a battery therewithin, with an electrode (204) connected to the pacing circuitry and protruding from a distal end of the inner cylindrical can (214).

5. The biostimulator of claim 4, wherein the longitudinal guide (205) of the housing (201) comprises longitudinal tracks in an outer surface of the inner cylindrical can (214).

6. The biostimulator of claim 5, wherein the frame (104) is sandwiched between the outer cylindrical can (202) and the inner cylindrical can (214), slidably engaging the longitudinal tracks.

7. The biostimulator of any one of claims 1 to 6, further comprising a retrieval button (102) protruding from a proximal end of the housing (201), the retrieval button (102) attached to or integral with the frame (104), wherein:
the biostimulator (100) is operable to have a first arrangement, for implantation, comprising the retrieval button (102) depressed to the proximal end of the housing (201), and the fixation tines (106) extended from a distal end of the housing (201); and
the biostimulator (100) is operable to have a second arrangement, for retrieval from implantation, comprising the retrieval button (102) withdrawn from the proximal end of the housing (201), and the fixation tines (106) retracted into the distal end of the housing (201).

8. The biostimulator of any one of the claims 1 to 7, wherein
the frame (104) has a plurality of longitudinal members each having a respective one of the fixation tines (106) at a distal end of the longitudinal member; and
the one or more circumferential bands or rings (208, 212) join and support the plurality of longitudinal members.

9. The biostimulator of any one of claims 1 to 6, further comprising:
a retrieval button (102) protruding from a proximal end of the housing (201), wherein the frame (104) and the fixation tines (106) are a unitary body comprising a memory material.

10. The biostimulator of any one of claims 1 to 9, wherein
the housing (201) has one or more locking channels (508) coupled to the longitudinal guide (205); and
the frame (104) engageable to the one or more locking channels (508) through rotation of the frame (104).

11. A biostimulator system, comprising:
a biostimulator transport system (300) comprising a handle (302), and an elongated catheter (304) extending from the handle (302) to a distal catheter end (306); and
the biostimulator (100) of any one of claims 1-10 releasably mountable on the distal catheter end (306) of the biostimulator transport system (300).

12. The biostimulator system of claim 11, wherein:
the biostimulator transport system (300) and the biostimulator (100) are operable to have a first arrangement, comprising the biostimulator (100) releasably mounted on the distal catheter end (306) of the biostimulator transport system (300), and the fixation tines (106) retracted into a distal end of the housing (201); and
the biostimulator transport system (300) and the biostimulator (100) are operable to have a second arrangement, comprising the fixation tines (106) extended from the distal end of the housing (201).

13. The biostimulator system of claim 11 or 12, further comprising:
the housing (210) having at least one locking channel (508); and
the frame (104) engageable to the at least one locking guide, through rotating a portion of the biostimulator transport system (300) when coupled to the frame (104).

14. A biostimulator system, comprising:
a biostimulator retrieval system (401) comprising a handle (302), an elongated catheter (304) extending from the handle (302) to a distal catheter end (306), and a snare (402) extending through the elongated catheter (304); and
the biostimulator (100) of any one of claims 1-10 arranged for grasping by the distal catheter end (306), for retrieval of the biostimulator.

15. The biostimulator system of claim 14, wherein
the housing (201) has at least one locking channel (508); and
the frame (104) engageable to the at least one locking channel (508), through rotating a portion of the biostimulator retrieval system (401) when coupled to the frame (104).

## Patentansprüche

1. Biostimulator (100), umfassend:
ein Gehäuse (201), beinhaltend ein Elektronikfach (203), enthaltend eine Schrittmacherschaltung, wobei das Gehäuse (201) einen Längskanal (205) und einen oder mehrere Umlaufkanäle (206, 201) beinhaltet; und
einen Rahmen (104), der bewegbar in den Längskanal (205) eingreift und ein/einen oder mehrere Umlaufbänder oder -ringe (208, 212) aufweist, die jeweils in einen jeweiligen Umlaufkanal (206, 210) beschränkt sind und in diesen eingreifen, wobei der Rahmen (104) eine Vielzahl von Fixierungszinken (106) an einem distalen Ende des Rahmens (104) aufweist und der Längskanal (205) einen Freiraum aufweist, um es dem Rahmen (104) zu ermöglichen, sich umlaufend innerhalb des Längskanals (205) zu bewegen, wenn sich das/der eine oder die mehreren Umlaufbänder oder -ringe (208, 212) in Bezug auf das Gehäuse (201) innerhalb des einen oder der mehreren Umlaufkanäle (206, 210) drehen.

2. Biostimulator nach Anspruch 1, wobei das Gehäuse (201) eine Vielzahl von Anschlägen (206, 210) aufweist.

3. Biostimulator nach Anspruch 2, wobei der Rahmen (104) dazu angeordnet ist, in die Vielzahl von Anschlägen (206, 210) einzugreifen, um eine Erstreckung und einen Einzug der Fixierungszinken (106) zu beschränken.

4. Biostimulator nach einem der Ansprüche 1 bis 3, wobei das Gehäuse (201) einen äußeren zylindrischen Behälter (202) und einen inneren zylindrischen Behälter (214) aufweist, der eine Batterie innerhalb dessen aufweist, wobei eine Elektrode (204) mit der Schrittmacherschaltung verbunden ist und von einem distalen Ende des inneren zylindrischen Behälters (214) vorsteht.

5. Biostimulator nach Anspruch 4, wobei die Längsführung (205) des Gehäuses (201) Längsschienen in einer äußeren Oberfläche des inneren zylindrischen Behälters (214) umfasst.

6. Biostimulator nach Anspruch 5, wobei der Rahmen (104) zwischen dem äußeren zylindrischen Behälter (202) und dem inneren zylindrischen Behälter (214) eingefügt ist, der verschiebbar in die Längsschienen eingreift.

7. Biostimulator nach einem der Ansprüche 1 bis 6, ferner umfassend eine Entnahmetaste (102), die von einem proximalen Ende des Gehäuses (201) vorsteht, wobei die Entnahmetaste (102) an dem Rahmen (104) angebracht oder mit diesem einstückig ist, wobei:
der Biostimulator (100) dazu betreibbar ist, eine erste Anordnung zur Implantation aufzuweisen, umfassend die Entnahmetaste (102), die zu dem proximalen Ende des Gehäuses (201) eingedrückt ist, und die Fixierungszinken (106), die sich von einem distalen Ende des Gehäuses (201) erstrecken; und
der Biostimulator (100) dazu betreibbar ist, eine zweite Anordnung zur Entnahme aus der Implantation aufzuweisen, umfassend die Entnahmetaste (102), die aus dem proximalen Ende des Gehäuses (201) zurückgezogen ist, und die Fixierungszinken (106), die in das distale Ende des Gehäuses (201) eingezogen sind.

8. Biostimulator nach einem der Ansprüche 1 bis 7, wobei
der Rahmen (104) eine Vielzahl von Längselementen aufweist, die jeweils eine jeweilige der Fixierungszinken (106) an einem distalen Ende des Längselements aufweisen; und
das/der eine oder die mehreren Umlaufbänder oder -ringe (208, 212) die Vielzahl von Längselementen verbinden und stützen.

9. Biostimulator nach einem der Ansprüche 1 bis 6, ferner umfassend:
eine Entnahmetaste (102), die von einem proximalen Ende des Gehäuses (201) vorsteht, wobei der Rahmen (104) und die Fixierungszinken (106) ein einheitlicher Körper sind, umfassend ein Speichermaterial.

10. Biostimulator nach einem der Ansprüche 1 bis 9, wobei
das Gehäuse (201) einen oder mehrere Verriegelungskanäle (508) aufweist, die an die Längsführung (205) gekoppelt sind; und
der Rahmen (104) durch eine Drehung des Rahmens (104) in den einen oder die mehreren Verriegelungskanäle (508) eingreifbar ist.

11. Biostimulatorsystem, umfassend:
ein Biostimulatortransportsystem (300), umfassend einen Griff (302) und einen länglichen Katheter (304), der sich von dem Griff (302) zu einem distalen Katheterende (306) erstreckt; und
den Biostimulator (100) nach einem der Ansprüche 1-10, der an dem distalen Katheterende (306) des Biostimulatortransportsystems (300) lösbar montierbar ist.

12. Biostimulatorsystem nach Anspruch 11, wobei:
das Biostimulatortransportsystem (300) und der Biostimulator (100) dazu betreibbar sind, eine erste Anordnung aufzuweisen, umfassend den Biostimulator (100), der an dem distalen Katheterende (306) des Biostimulatortransportsystems (300) lösbar montiert ist, und die Fixierungszinken (106), die in ein distales Ende des Gehäuses (201) eingezogen sind; und
das Biostimulatortransportsystem (300) und der Biostimulator (100) dazu betreibbar sind, eine zweite Anordnung aufzuweisen, umfassend die Fixierungszinken (106), die sich von dem distalen Ende des Gehäuses (201) erstrecken.

13. Biostimulatorsystem nach Anspruch 11 oder 12, ferner umfassend:
das Gehäuse (210), das mindestens einen Verriegelungskanal (508) aufweist; und
den Rahmen (104), der durch eine Drehung eines Abschnitts des Biostimulatortransportsystems (300), wenn an den Rahmen (104) gekoppelt, in die mindestens eine Verriegelungsführung eingreifbar ist.

14. Biostimulatorsystem, umfassend:
ein Biostimulatorentnahmesystem (401), umfassend einen Griff (302), einen länglichen Katheter (304), der sich von dem Griff (302) zu einem distalen Katheterende (306) erstreckt, und eine Schlinge (402), die sich durch den länglichen Katheter (304) hindurch erstreckt; und
den Biostimulator (100) nach einem der Ansprüche 1-10, der zum Greifen durch das distale Katheterende (306) zur Entnahme des Biostimulators angeordnet ist.

15. Biostimulatorsystem nach Anspruch 14, wobei
das Gehäuse (201) mindestens einen Verriegelungskanal (508) aufweist; und
der Rahmen (104) durch eine Drehung eines Abschnitts des Biostimulatorentnahmesystems (401), wenn an den Rahmen (104) gekoppelt, in den mindestens einen Verriegelungskanal (508) eingreifbar ist.

## Revendications

1. Biostimulateur (100), comprenant :
un logement (201) incluant un compartiment électronique (203) contenant un circuit de stimulation, dans lequel le logement (201) inclut un canal longitudinal (205) et un ou plusieurs canaux circonférentiels (206, 201) ; et
un cadre (104) mis en prise de façon mobile avec le canal longitudinal (205) et ayant une ou plusieurs bandes ou bagues circonférentielles (208, 212) chacune contrainte dans un canal circonférentiel (206, 210) respectif et venant en prise avec celui-ci, dans lequel le cadre (104) a une pluralité de pointes de fixation (106) au niveau d'une extrémité distale du cadre (104), et le canal longitudinal (205) a un espace libre pour permettre au cadre (104) de se déplacer de façon circonférentielle à l'intérieur du canal longitudinal (205) lorsque les une ou plusieurs bandes ou bagues circonférentielles (208, 212) tournent par rapport au logement (201) à l'intérieur des un ou plusieurs canaux circonférentiels (206, 210).

2. Biostimulateur selon la revendication 1, dans lequel le boîtier (201) comporte une pluralité d'arrêt (206, 210).

3. Biostimulateur selon la revendication 2, dans lequel le cadre (104) est agencé pour s'engager avec la pluralité d'arrêt (206, 210) de manière à limiter l'extension et la retrait des tines de fixation (106).

4. Biostimulateur selon l'une quelconque des revendications 1 à 3, dans lequel le boîtier (201) comporte un boîtier cylindrique extérieur (202), et un boîtier cylindrique intérieur (214) comportant une batterie à l'intérieur de celui-ci, avec une électrode (204) connectée au circuit de stimulation et faisant saillie depuis une extrémité distale du boîtier cylindrique intérieur (214).

5. Biostimulateur selon la revendication 4, dans lequel le guide longitudinal (205) du boîtier (201) comprend des pistes longitudinales dans une surface extérieure du bombe cylindrique intérieur (214).

6. Biostimulateur selon la revendication 5, dans lequel le cadre (104) est pris en sandwich entre le bombe cylindrique extérieur (202) et le bombe cylindrique intérieur (214), s'engager de manière coulissante dans les pistes longitudinales.

7. Biostimulateur selon l'une quelconque des revendications 1 à 6, comprenant en outre un bouton de récupération (102) faisant saillie depuis une extrémité proximale du boîtier (201), le bouton de récupération (102) étant fixé au cadre (104) ou solidaire de celui-ci, dans lequel :
le biostimulateur (100) peut fonctionner pour avoir un premier agencement, pour l'implantation, comprenant le bouton de récupération (102) pressé vers l'extrémité proximale du boîtier (201), et les tines de fixation (106) prolongées à partir d'une extrémité distale du boîtier (201) ; et
le biostimulateur (100) peut fonctionner pour avoir un second agencement, pour la récupération de l'implantation, comprenant le bouton de récupération (102) retiré de l'extrémité proximale du boîtier (201), et les tines de fixation (106) rétracté dans l'extrémité distale du boîtier (201).

8. Biostimulateur selon l'une quelconque des revendications 1 à 7, dans lequel
le cadre (104) comporte une pluralité de éléments longitudinaux ayant chacun une des dents de fixation (106) respectives à une extrémité distale du élément longitudinal ; et
les une ou plusieurs bandes ou anneaux circonférentiels (208, 212) rejoignent et supportent la pluralité de longerons.

9. Biostimulateur selon l'une quelconque des revendications 1 à 6, comprenant en outre :
un bouton de récupération (102) faisant saillie depuis une extrémité proximale du boîtier (201), dans lequel le cadre (104) et les tines de fixation (106) sont un corps unitaire comprenant un matériau mémoire.

10. Biostimulateur selon l'une quelconque des revendications 1 à 9, dans lequel :
le boîtier (201) comporte un ou plusieurs canaux de verrouillage (508) couplés au guide longitudinal (205) ; et
le cadre (104) pouvant s'engager avec les un ou plusieurs canaux de verrouillage (508) par rotation du cadre (104).

11. Système de biostimulateur, comprenant :
un système de transport de biostimulateur (300) comprenant une poignée (302), et un cathéter allongé (304) s'étendant de la poignée (302) à une extrémité de cathéter distale (306) ; et
le biostimulateur (100) selon l'une quelconque des revendications 1 à 10 monté de façon amovible sur l'extrémité de cathéter distale (306) du système de transport de biostimulateur (300).

12. Système de biostimulateur selon la revendication 11, dans lequel :
le système de transport de biostimulateur (300) et le biostimulateur (100) peuvent fonctionner pour avoir un premier agencement, comprenant le biostimulateur (100) monté de manière amovible sur l'extrémité de cathéter distale (306) du système de transport de biostimulateur (300), et les tines de fixation (106) rétracté dans une extrémité distale du boîtier (201) ; et
le système de transport de biostimulateur (300) et le biostimulateur (100) peuvent fonctionner pour avoir un second agencement, comprenant les tines de fixation (106) prolongées à partir de l'extrémité distale du boîtier (201).

13. Système de biostimulateur selon la revendication 11 ou 12, comprenant en outre :
le boîtier (210) comportant au moins un canal de verrouillage (508) ; et
le cadre (104) pouvant s'engager avec l'au moins un guide de verrouillage, à travers la rotation d'une partie du système de transport de biostimulateur (300) lorsqu'il est couplé au cadre (104).

14. Système de biostimulateur, comprenant :
un système de récupération de biostimulateur (401) comprenant une poignée (302), un cathéter allongé (304) s'étendant de la poignée (302) à une extrémité de cathéter distale (306), et un snare (402) s'étendant à travers le cathéter allongé (304) ; et
le biostimulateur (100) selon l'une quelconque des revendications 1 à 10, agencé pour être saisi par l'extrémité de cathéter distale (306), pour la récupération du biostimulateur.

15. Système de biostimulateur selon la revendication 14, dans lequel :
le boîtier (201) comporte au moins un canal de verrouillage (508) ; et
le cadre (104) pouvant s'engager avec l'au moins un canal de verrouillage (508), en faisant tourner une partie du système de récupération de biostimulateur (401) lorsqu'il est couplé au cadre (104).
